(19) Europäisches Patentamt
European Patent Office
Office européen des brevets

(11) Publication number: **0 193 307**
**B1**

(12) **EUROPEAN PATENT SPECIFICATION**

(45) Date of publication of patent specification: 09.08.89

(21) Application number: 86300955.1

(22) Date of filing: 12.02.86

(51) Int. Cl.[4]: C 22 B 3/00, C 22 B 15/12, C 22 B 19/26, C 07 D235/06, C 07 D233/60, C 07 D233/61

(54) **Process for the extraction of metal values and novel metal extractants.**

(30) Priority: 25.02.85 GB 8504818

(43) Date of publication of application: 03.09.86 Bulletin 86/36

(45) Publication of the grant of the patent: 09.08.89 Bulletin 89/32

(84) Designated contracting states: AT BE CH DE FR GB IT LI LU NL SE

(56) References cited:
EP-A- 0 057 797
AT-B- 342 616
DE-A- 1 803 728
DE-A- 2 553 021
DE-A- 2 625 839
DE-B- 1 091 562
FR-A- 2 276 821
FR-A- 2 302 732
US-A- 3 843 667
US-A- 4 041 019
US-A- 4 189 543
CHEMICAL ABSTRACTS, vol. 74, no. 13, 29th March 1971, page 306, column 2, abstract no. 64248x, Columbus, Ohio, US; & JP - B - 45 31174
CHEMISTRY AND INDUSTRY, no. 2, 19th January 1980, pages 85, 86; M. OGATA et al.: "Reaction of N, N'-carbonyldiimidazole and N,N'-thionyldimidazole with alcohols: an imidazole transfer reaction"
TETRAHEDRON, vol. 39, no. 22, 1983, pages 3797-3800; A. McKILLOP et al.: "4- and 5-nitroimidazoles: 13C NMR assignment of structure"

(73) Proprietor: IMPERIAL CHEMICAL INDUSTRIES PLC
Imperial Chemical House Millbank
London SW1P 3JF (GB)

(72) Inventor: Devonald, David Phillip
134 Stockfield Mount
Oldham Lancs OL9 9AS (GB)
Inventor: Nelson, Anthony John
65 Evesham Road Alkrington
Middleton Manchester M24 1QL (GB)
Inventor: Robinson, Frank
32 Springside View Brandlesholme
Bury Lancs BL8 4LU (GB)
Inventor: Quan, Peter Michael
23 Hawthorn Road
Rochdale Lancs OL11 55Q (GB)
Inventor: Steward, David
21 Clifton Crescent Heyside
Royton Lancs OL2 65F (GB)

(74) Representative: Beton, John Lonsdale et al
Imperial Chemical Industries PLC Legal Department: Patents PO Box 6
Welwyn Garden City Herts, AL7 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

EP 0 193 307 B1

ROCZNIKI CHEMII ANN. SOC. CHIM. POLONORUM, vol. 46, 1972, pages 717-729; T. LESIAK et al.: "New derivatives of carbamic acid and urea"
ANNALEN DER CHEMIE, vol. 648, 1961, pages 72-82; H.A. STAAB et al.: "Reaktionsfähige heterocyclische Amide der Kohlensäure, VI. Synthese und Eigenschaften von N-Carbonsäureamiden der Azole eine neue Isocyanat-Synthese"
CHEMICAL ABSTRACTS, vol. 101, no. 1, 2nd July 1984, page 612, column 1, abstract no. 7152p, Columbus, Ohio, US
JOURNAL OF THE AMERICAN CHEMICAL SOCIETY, vol. 71, July 1949, pages 2297-2300; R.A. HENRY et al.: "Aromatic isocyanates as reagents for the identification of some heterocyclic compounds"
CHEMICAL ABSTRACTS, vol. 74, no. 13, 29th March 1971, page 306, column 2, abstract no. 64248x, Columbus, Ohio, US; & JP - B - 45 31174
CHEMISTRY AND INDUSTRY, no. 2, 19th January 1980, pages 85, 86; M. OGATA et al.: "Reaction of N, N'-carbonyldiimidazole and N,N'-thionyldimidazole with alcohols: an imidazole transfer reaction"

**Description**

This invention relates to a process for the extraction of metal values from aqueous solutions of metal salts, and in particular to a process for the extraction of metal values from aqueous solutions in the presence of halide anions.

The use of solvent extraction techniques for the hydrometallurgical recovery of metal values from metal ores has been practised commercially for a number of years. For example copper may be recovered from oxide ores or from ore tailings by treating the crushed ore with sulphuric acid to give an aqueous solution of copper sulphate which is subsequently contacted with a solution in a water-immiscible organic solvent of a metal extractant whereby the copper values are selectively extracted into the organic phase.

The application of solvent extraction techniques to aqueous solutions containing halide anions however has presented numerous technical problems. For example copper bearing sulphur-containing ores such as chalcopyrite may be leached using ferric chloride or cupric chloride solutions, but the solvent extraction of the resultant leach solutions presents formidable difficulties. The use of imidazoles for recovering metals by solvent extraction from halide-containing solutions is known from DE-A-2 625 839. The recovery of zinc by solvent extraction from halide-containing solutions such as those derived from sulphur-containing ores by chloride leaching has also been proposed (See for example, G. M. Ritcey, B. H. Lucas and K. T. Price, Hydrometallurgy, 1982, 8, page 197). However, known extractants for zinc (for example organophosphorous compounds such as tributyl phosphate) generally show a poor efficiency of metal recovery and a poor selectivity for zinc over the iron present in such leach solutions.

The present invention provides a process for the extraction of metal values from aqueous solutions containing halide or pseudohalide ions by the use of metal extractants whose several properties meet the stringent requirements imposed on the extractant by the system.

According to the present invention there is provided a process for extracting metal values from aqueous solutions of metal salts containing halide or pseudo halide anions which comprises contacting the aqueous solution with a solution in a water-immiscible organic solvent of a N-substituted 1,3-diazole compound of formula :

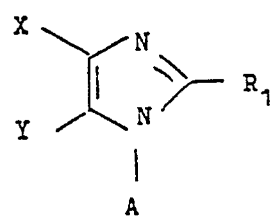

(i)

wherein

$R_1$ is hydrogen or an optionally substituted lower alkyl group or an optionally substituted vinyl group ;

X and Y, which may be the same or different, are separately hydrogen, an optionaly substituted hydrocarbon group a halogen, a nitro group, a cyano group or a carboxylic ester group, or where X and Y both together with the two carbon atoms joining them form an optionally substituted aromatic or aliphatic cyclic group ; and

A is the group $—C=O \cdot OR_2$, $—C=O \cdot R_2$, $—SO_2R_2$ or $—C=O \cdot NR_3R_4$ wherein $R_2$ is a hydrocarbyl group containing from 5 to 36 carbon atoms and $R_3$ and

$R_4$ are separately hydrogen or a hydrocarbyl group, $R_3$ and $R_4$ together containing from 5 to 36 alkyl carbon atoms.

As diazole compounds which may be used in the present invention there may be mentioned benzimidazoles of formula :

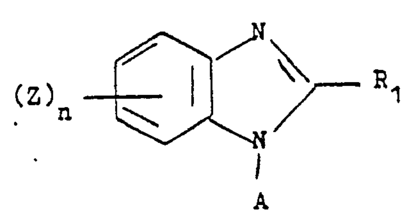

(ii)

wherein $—(Z)_n$ represents n optional substituents Z and n is from 0 to 3 ; imidazoles of formula :

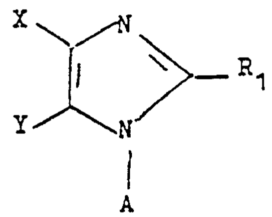

(iii)

and tetrahydrobenzimidazoles of formula :

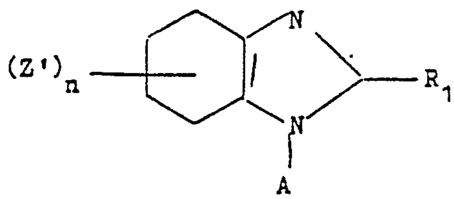

(iv)

wherein $(Z')_n$ represents n substituents Z' wherein n is from 0 to 3.

Each Z (and Z') may separately be for example a hydrocarbyl group (for example a lower alkyl group), halogen, a nitro group, a cyano group or a carboxylic ester group. In the tetrahydrobenzimidazole, it is preferred that Z' (if present) is a hydrocarbyl group. The number, n, of substituents Z (or Z') is preferably 0, 1 or 2.

$R_1$ is preferably hydrogen, as we have found that such compounds generally have a suitable strength of ligand and a better selectivity for zinc over acid (as will be discussed later).

It is preferred that the total number of alkyl carbon atoms in all substituents of the diazole compound is from 9 to 30 and preferably from 14 to 30, since the solubility of the compound in suitable organic solvents may be undesirably low if the total number of saturated carbon atoms is less than 14, whilst the disengagement of the organic and aqueous phases during the solvent extraction may be undesirably slow if the total number of alkyl carbon atoms exceeds 30.

$R_2$ in the group A ($—C = O \cdot R_2$, $—SO_2R_2$ or $—C = O \cdot R_2$) may for example be an alkyl group, for example an octyl, nonyl, decyl, dodecyl, tridecyl, pentadecyl, hexadecyl or octadecyl group or a higher alkyl group. $R_2$ may for example be a cyclo alkyl group such as cyclohexyl. $R_2$ may for example be an aryl, or more preferably alkylaryl or alkoxyaryl group.

To achieve good solubility of the compound in preferred organic solvents, $R_2$ is preferably a branched alkyl group or a mixture (including an isomeric mixture) of branched alkyl groups. Similarly, if $R_2$ is an aralkyl or alkoxyaryl group, the alkyl portion of the group is preferably a branched alkyl group or a mixture (including an isomeric mixture) of branched alkyl groups. The preferred number of alkyl carbon atoms in the group $R_2$ depends on the total number of alkyl carbon atoms in the other substituents of the diazole compound. For example if $R_1$ is hydrogen or methyl and there is used a benzimidazole compound whose substituent(s) Z together contain either no alkyl carbon atoms or at most one alkyl carbon atom or there is used an imidazole whose substituents X and Y together contain either no alkyl carbon atoms or at most one alkyl carbon atom or there is used a tetrahydrobenzimidazole whose substituent(s) Z together contain either no alkyl carbon atoms or at most one alkyl carbon atom, then in each case it is preferred that $R_2$ contains from 9 to 24 carbon atoms.

Compounds for use in the present invention wherein —A is the group $—C = O \cdot R_2$ may be prepared by acylation of the appropriate imidazole, benzimidazole or tetrahydrobenzimidazole using for example an acid chloride of formula $R_2—C = O \cdot Cl$. The acid chloride may itself be prepared by oxidation and chlorination of the alcohol $R_2CH_2OH$.

Compounds for use in the present invention wherein —A is the group $—C = O \cdot OR_2$ may be prepared for example from the alcohol $R_2OH$ by treatment with phosgene to give the chloroformate compound $R_2O—C = O \cdot Cl$ which may be reacted with the appropriate imidazole, benzimidazole or tetrahydrobenzimidazole to give the desired product.

Compounds for use in the present invention wherein —A is the group $—SO_2R_2$ may be prepared by treatment of the appropriate imidazole, benzimidazole or tetrahydrobenzimidazole with a hydrocarbyl sulphonyl chloride $R_2SO_2Cl$ which may be obtained from the corresponding sulphonic acid.

Highly branched groups $R_2$ may be usefully derived from branched alcohols prepared by the Guerbet and Aldol condensations. Such alcohols are characterised by branching at the position beta to the hydroxyl group and have the general formula :

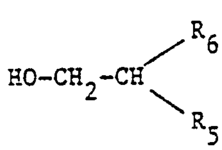

(v)

wherein $R_5$ and $R_6$ are both alkyl groups and $R_5$ contains two fewer carbon atoms than $R_6$. $R_5$ and $R_6$ may be straight chain or branched chain alkyl groups and may be isomeric mixtures of alkyl groups. A mixture of highly branched alcohols may be obtained by Guerbet or Aldol condensations of mixtures of alcohols and aldehydes respectively. By way of Example, good solubility in preferred organic solvents is conferred on diazole compounds of formula (i) above wherein the group —A is the group $—C=O \cdot OR_2$, $—C=O \cdot R_2$ or $—SO_2R_2$ and $R_2$ is derived from commercial isooctadecanol prepared by the aldol dimerisation of commercial nonanol and believed to consist essentially of a mixture of geometrical isomers of the compound (vi) :

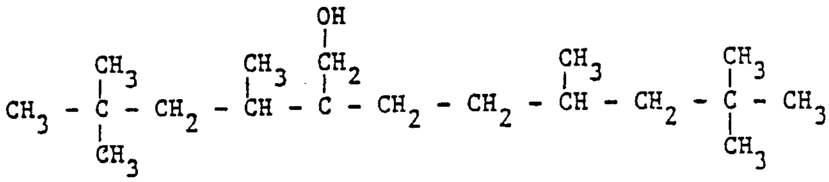

It will be noted that using the reaction scheme proposed above, isooctadecanol ($C_{17}H_{35} \cdot CH_2OH$ as above) for example gives rise to the group $—C=O \cdot C_{17}H_{35}$ on acylation of the diazole with the acid chloride derived from the alcohol. This group has a high degree of branching at the position adjacent to the carbonyl function in the group $—C=O \cdot R_2$. In contrast, when the group $—C=O \cdot OR_2$ is prepared by the reaction of phosgene with the alcohol to form the chloroformate, isooctadecanol ($—C=O \cdot OC_{17}H_{35} \cdot CH_2OH$ as above) for example gives rise to the group $—C=O \cdot CH_2C_{17}H_{35}$ in which the position adjacent to the carbonyl group is occupied by the $—CH_2$ group and the branching only occurs at the next adjacent carbon atom.

We have found that compounds having a high degree of branching, and especially compounds having a high degree of branching at the position adjacent to the carbonyl function in the group —A, show an improved resistance to hydrolysis under the stringent conditions employed in the solvent extraction process.

Thus when —A is $—C=O \cdot R_2$, the group $R_2$ may, as noted above, be derived from the primary alcohol $R_2CH_2OH$. Primary alcohols having a high degree of branching at the carbon atom adjacent to the $—CH_2OH$ group, for example alcohols such as the isooctadecyl alcohol of formula (vi) above, give rise to a highly branched group $R_2$ adjacent to the carbonyl function and are especially preferred when —A is of the formula $—C=O \cdot R_2$.

As noted above, when —A is of the formula $—C=O \cdot OR_2$, and is prepared by the reaction of the alcohol and phosgene to form the chloroformate, primary alcohols such as isooctadecanol having a high degree of branching at the carbon atom adjacent to the $—CH_2OH$ group give rise to a product in which the highly branched group is separated from the carbonyl function by the $—CH_2—$ group. Whilst we have found that such products still have superior hydrolytic stability as compared with the corresponding unbranched compound, further improvements in hydrolytic stability may generally be obtained by using a secondary alcohol $R_2OH$, for example 3,9-diethyl-tridecan-6-ol as starting material. Such a secondary alcohol provides the branching in a position directly adjacent to the carbonyl function.

The diazoles for use in the present invention wherein —A is the group $—C=O \cdot NR_3R_5$ may be prepared by conventional means, for example by treatment of the appropriate benzimidazole, imidazole or tetrahydrobenzimidazole with an alkyl isocyanate in the presence of a catalyst such as dibutyltin dilaurate.

The amide group $—NR_3R_4$ may be secondary ($R_3$ is hydrogen) or tertiary. $R_3$ and $R_4$, which may be the same or different, may be groups of the type indicated above for $R_2$. $R_3$ and $R_4$ taken together contain from 5 to 36 carbon atoms. Thus $R_3$ may be for example a lower alkyl group, for example a methyl group, provided $R_4$ is correspondingly larger. $R_3$ and $R_4$ taken together are preferably alkyl groups containing a total of from 15 to 36 carbon atoms. For tertiary amides sufficient solubility in preferred organic solvents may generally be achieved if $R_3$ and $R_4$ are straight chain or branched chain alkyl groups. However for secondary amides ($R_3$ is hydrogen), $R_4$ is preferably a branched chain alkyl group.

The process of the present invention may be applied to the extraction from aqueous solutions containing halide or pseudohalide ion of any metal capable of forming a stable halide or pseudohalide containing complex with the diazole compound in the water-immiscible organic solvent. Examples of such metals include copper, cobalt, cadmium and zinc. The process of the present invention is especially suitable for the solvent extraction of copper and zinc from aqueous solution obtained by the halide or pseudohalide leaching of sulphur containing ores. In general, such ores contain both copper and zinc in relative proportions which vary from ore to ore. It is convenient to recover both copper and zinc in successive processing stages from the leach solutions. For example the copper may be recovered from the leach solution by solvent extraction and the raffinate from this process may be treated in a separate solvent extraction process for the recovery of zinc. The process of the present invention may be used to recover either copper or zinc, although the same diazole compound will not necessarily be used as the extractant in both cases. Alternatively, the process of the present invention may be used for example in

the zinc extraction stage only and a different solvent extractant (for example a solvent extractant disclosed in European Patent Application 0 057 797) used in the copper extraction stage. Alternatively again, the process of the present invention may be used for the extraction of copper and a different process used for the recovery of any other metals which it may be desired to extract from the feed.

It will be appreciated that the process of the present invention may be incorporated into a wide variety of different methods for the overall recovery of metals from their ores or from other metal-bearing sources. Details of these methods will vary depending on the metal concerned and the nature and composition of the leach solution. By way of example, an integrated process which is especially suitable for leach solutions containing high levels of cupric ion is described in European Patent Application No 0 57 797.

The diazole compounds for use in the present invention are especially useful for the recovery of zinc which has hitherto proved to be very difficult to recover effectively by solvent extraction. Thus the compounds of the present invention have in general a high affinity for zinc, which in general is combined with an excellent selectivity for zinc over the iron which is inevitably present in the leach solution especially for example when ferric chloride is used as leachant. However, even compounds which have a high affinity for zinc also retain a high affinity for copper, and such reagents are not selective for zinc in the presence in high levels of copper. This is not a significant disadvantage in practice even when it is desired to recover zinc from a solution containing both copper and zinc, since the recovery of zinc generally takes place after the bulk of copper has been removed, for example in a first solvent extraction stage. In fact, it is by no means essential for the zinc extractant to show any degree of selectivity over copper at all. Thus it is perfectly feasible to remove residual copper remaining in the raffinate, for example by cementation using a metal such as zinc or iron, and to treat the resulting copper-free aqueous solution by solvent extraction to recover the zinc.

The zinc solvent extraction circuit may be similar in design to that proposed in European Patent Application 0 057 797 for the recovery of copper from halide containing solutions by solvent extraction. Thus for example in a circuit for the recovery of copper and zinc from the aqueous leach solution derived from the leaching of a sulphur-containing ore with for example ferric chloride, the aqueous copper-free raffinate from the copper solvent extraction/cementation stage will contain zinc, iron and halide ion (for example 35 gpl zinc, 70 gpl iron, 3.7 M in chloride ion and containing 5 gpl hydrochloric acid). This feed to the zinc circuit may be contacted with a solution of the extractant of the present invention in a water immiscible organic solvent into which the zinc is extracted. The loaded organic phase solution is contacted with an aqueous strip solution containing a reduced level of zinc and halide ion such that at least a proportion of the zinc transfers into the aqueous strip phase. The stripped organic phase is returned to extract more zinc, and the loaded aqueous strip solution is passed to a zinc recovery stage, typically an electrowinning stage. The electrowinning stage may produce metallic zinc and chlorine gas (as described for example in « Zinc Electrowinning from Chloride Electrolyte » by D. J. MacKinnon and J. M. Brannen; Mining Engineering April 1982 page 409) which may be used to regenerate the ferric chloride leachant (now reduced to ferrous ion). Alternatively an internal regeneration of the leachant may take place in a split cell without the generation of free chlorine gas. The zinc and chloride ion depleted aqueous stream from the electrowinning stage is returned to the strip stage to act as the aqueous strip solution, thereby completing the zinc extraction circuit.

The extraction process of the present invention may be represented by an equation such as the following:

$$2L_{org} + M^{++}_{aq} + 2Cl^{-}_{aq} \rightleftharpoons (L_2MCl_2)_{org}$$

where M is a divalent metal ion such as copper or zinc.

This equation ia a grossly oversimplified representation of a very complex process and is not to be taken as in any way limiting the scope of the present invention, but it serves to illustrate the formation of a neutral organic phase complex of the divalent metal and the extractant (L) which is believed to predominate in the process of the present invention. The equation illustrates the reversible nature of the extraction, whereby the complex of the metal and the extractant in the organic phase can be stripped on contact with the aqueous solution from the electrowinning stage which is depleted in the metal and in the halide ion.

A further property which is of importance for an extractant in the process of the present invention is the absence of significant protonation by the acidic leach liquor. Such protonation may be represented by an equation such as:

$$L_{org} + H^{+}_{aq} + Cl^{-}_{aq} \rightleftharpoons (LH^{+}Cl^{-})_{org}$$

where L is the extractant. Such protonation of the ligand carries hydrochloric acid into the organic phase and builds up an excessive chloride ion concentration on the strip side. We have found that this problem is particularly acute for the extraction of zinc which is thought to promote the acid transfer. Preferred reagents of the present invention combine a high affinity for zinc with a low acid transfer into the organic phase.

As illustrated by the Examples, the extractants of the present invention provide a range of properties so that the optimum extractant may be selected for a given leach solution. Thus for example, the extractants of Examples 3, 4, 10 and 11 are relatively poor extractants for zinc, and would be used in preference for the extraction of copper. Furthermore « strong » extractants such as that of Example 12 are used most effectively in removing copper or zinc from dilute solutions low in chloride ion concentration where the high affinity for the metal is of advantage. In solutions of higher concentration of acid and chloride ion, the ligand would tend to transfer an unacceptably high level of acid. In general we have found that an excellent balance of properties including ligand strength an low acid transfer, especially in the extraction of zinc may be obtained using benzimidazoles of formula (ii) wherein n is 0 and —A is $—C=O \cdot OR_2$. Such compounds also generally show good solubility in preferred organic solvents, and superior hydrolytic stability relative to the other compounds of the invention.

Examples of suitable water-immiscible organic solvents are aliphatic, aromatic and alicyclic hydrocarbons, chlorinated hydrocarbons such as perchloroethylene, trichloroethane and trichloroethylene. Mixtures of solvents may be used. Especially preferred in conventional hydrometallurgical practice are mixed hydrocarbon solvents such as high boiling, high flash point petroleum fractions (for example kerosene) with varying aromatic content. In general, hydrocarbon solvents having a high aromatic content, for example AROMASOL H which consists essentially of a mixture of trimethylbenzenes and commercially available from Imperial Chemical Industries PLC (AROMASOL is a trade mark) or SOLVESSO 150 commercially available from Esso (SOLVESSO is a trade mark), provide a higher solubility for the extractant and its metal complex, whilst kerosene having a relatively low aromatic content, for example ESCAID 100 which is a petroleum distillate comprising 20 % aromatics, 56.6 % paraffins and 23.4 % naphthenes commercially available from ESSO (ESCAID is a trade mark) may in certain cases improve the hydrometallurgical performance of the extractant. Factors influencing the solubility of the extractant and its metal complex are complicated, but in general extractants having highly branched substituents and/or an isomeric mixture of substituents have comparatively high solubility. The concentration of the extractant in the water-immiscible organic solvent may be chosen to suit the particular leach solution to be treated. Typical values of extractant concentration in the organic phase are between about 0.1 to 2 Molar, and an especially convenient range is from 0.2 to 1.0 Molar in the organic solvent.

Certain diazoles for use in the present invention are novel compounds and the present invention includes such novel compounds as defined in claim 11.

This invention is illustrated by the following Examples in which all parts and percentages are by weight unless otherwise stated.

## Example 1

1-isooctadecanoylbenzimidazole was prepared as follows :

Commercial isooctadecanol was oxidised to the acid using potassium permanganate and converted to the acid chloride using thionyl chloride. Isooctadecanoyl chloride (45.38 g) was added dropwise over 5 minutes to a stirred solution of benzimidazole (36.12 g) in acetone (250 ml). After 30 minutes the mixture was filtered, and the solvent was removed under reduced pressure. Petroleum ether (boiling point 60° to 80 °C) (250 ml) was added and the solution was again filtered and washed with dilute hydrochloric acid followed by water. The product was dried over magnesium sulphate to give after removal of the petroleum ether 48.78 g of a viscous yellow oil. The product had a boiling point of 180° to 200 °C at 0.15 mm of mercury pressure, and its structure was confirmed by infrared and n.m.r. spectroscopy.

The ability of 1-isooctadecanoylbenzimidazole to extract copper from aqueous solution containing chloride ion was investigated by the following general method :

An aqueous solution was prepared which was 0.1 M in cupric chloride (6.35 gpl copper), and 0.1 M in hydrochloric acid and which contained 250 gpl of calcium chloride dihydrate. This solution was then agitated for 1.5 minutes with an equal volume of a solution which was a 0.2 M solution of the extractant in SOLVESSO 150. The layers were allowed to separate and settle, and were separately analysed for copper content. The transfer of copper from the aqueous to the organic phase was calculated as the percentage of the ligand taken up as the copper complex (assuming the complex $L_2CuCl_2$). The transfer of hydrochloric acid from the aqueous solution into the organic solution was calculated as the percentage of ligand that was protonated. The test was repeated using different molarities of hydrochloric acid and different concentrations of calcium chloride, and the results are presented in Table 1.

A similar test was used to determine the ability of the extractant to extract zinc from aqueous solution. An aqueous solution was prepared which was 0.6 M in zinc chloride (39.24 gpl zinc) and 0.01 M in hydrochloric acid and which contained 176.5 gpl of calcium chloride dihydrate (the calcium chloride concentration was adjusted to take account of the higher zinc chloride concentration). This solution was

then agitated for 1.5 minutes with an equal volume of a solution which was a 0.2 M solution of the extractant in SOLVESSO 150. The layers were allowed to separate and settle, and were separately analysed for zinc content. The transfer of zinc from the aqueous to the organic phase was calculated as the percentage of the ligand taken up as the zinc complex (assuming the complex $L_2ZnCl_2$). The transfer of hydrochloric acid from the aqueous solution into the organic solution was calculated as the percentage of ligand that was protonated. The test was repeated using different molarities of hydrochloric acid and different concentrations of calcium chloride, and the results are presented in Table 2. The results show that the ligand has an excellent affinity for zinc in the absence of acid, and is also a strong extractant for copper.

Example 2

1-isooctadecanoyl-5(6)-chlorobenzimidazole was prepared from 5(6)-chlorobenzimidazole (a tautomeric mixture of the 5-chloro- and 6-chloro- compounds) and isooctadecanoyl chloride using the method of Example 1. The structure was confirmed by infrared and n.m.r. analysis. 5(6)-Chlorobenzimidazole is prepared by the reaction of formic acid and 1-chloro-3,4-diaminobenzene in known manner.

The compound was evaluated as an extractant for copper and zinc using the procedure of Example 1, and the results are presented in Tables 1 and 2 respectively. The results show that the ligand has an excellent affinity for zinc in the absence of acid, and is also a strong extractant for copper.

Example 3

1-isooctadecanoyl-5(6)-nitrobenzimidazole was prepared from commercially available 5(6)-nitrobenzimidazole and isooctadecanoyl chloride using the method of Example 1. The structure was confirmed by infrared and n.m.r. analysis. The compound was evaluated as an extractant for copper and zinc using the procedure of Example 1, and the results are presented in Tables 1 and 2 respectively. The results show that the ligand has a good affinity for copper coupled with a very low acid transfer.

Example 4

1-isooctadecanoyl-2-methyl-5(6)-chlorobenzimidazole was prepared from 2-methyl-5(6)-chlorobenzimidazole and isooctadecanoyl chloride using the method of Example 1. The structure was confirmed by infrared and n.m.r. analysis. The 2-methyl-5(6)-chlorobenzimidazole was prepared in known manner from 1-chloro-3,4-diaminobenzene and acetic acid. The compound was evaluated as an extractant for copper and zinc using the procedure of Example 1, and the results are presented in Tables 1 and 2 respectively. The results show that the ligand has a good affinity for copper coupled with a very low acid transfer.

Example 5

1-isooctadecanoyl-2-methyl-benzimidazole was prepared from 2-methylbenzimidazole and isooctadecanoyl chloride using the method of Example 1. The structure was confirmed by infrared and n.m.r. analysis. The compound was evaluated as an extractant for copper and zinc using the procedure of Example 1, and the results are presented in Tables 1 and 2 respectively. The results show that the ligand has substantial affinity for zinc and good affinity for copper.

Example 6

1-isooctadecyloxycarbonyl-2-methyl-5(6)-chlorobenzimidazole was prepared as follows:

Isooctadecyl chloroformate (16.63 g) was added to a stirred mixture of 5(6)-chloro-2-methylbenzimidazole (9.16 g) and potassium carbonate (6.9 g) in acetone (100 ml). After 2 hours the mixture was filtered and the solvent removed from the filtrate. The residual oil was dissolved in petroleum ether (100 ml — b.p. 60 to 80 °C), filtered, and washed with dilute hydrochloric acid followed by water. Drying with magnesium sulphate and removal of the solvent gave 15.43 g of an amber viscous oil. The structure of the product was confirmed by infrared and n.m.r. analysis.

The compound was evaluated as an extractant for copper and zinc using the procedure of Example 1, and the results are presented in Tables 1 and 2 respectively. The results show that the ligand has good affinity for copper.

Example 7

1-isooctadecyloxycarbonyl-5(6)-chlorobenzimidazole was prepared from commercial isooctadecanol and 5(6)-chlorobenzimidazole using the method of Example 6. The structure was confirmed by infrared and n.m.r. analysis.

The compound was evaluated as an extractant for copper and zinc using the procedure of Example 1,

and the results are presented in Tables 1 and 2 respectively. The results show that the ligand has substantial affinity for zinc and good affinity for copper coupled with a low acid transfer.

Example 8

1-isooctadecyloxycarbonyl-2-methylbenzimidazole was prepared from commercial isooctadecanol and 2-methylbenzimidazole using the method of Example 6. The structure was confirmed by infrared and n.m.r. analysis.

The compound was evaluated as an extractant for copper and zinc using the procedure of Example 1, and the results are presented in Tables 1 and 2 respectively. The results show that the ligand has a substantial affinity for zinc and good affinity for copper coupled with low acid uptake.

Example 9

1-isooctadecyloxycarbonylbenzimidazole was prepared from commercial isooctadecanol and benzimidazole using the method of Example 6. The structure was confirmed by infrared and n.m.r. analysis.

The compound was evaluated as an extractant for copper and zinc using the procedure of Example 1, and the results are presented in Tables 1 and 2 respectively. The results show that the ligand has excellent affinity for both zinc and copper coupled with a very low acid transfer.

Example 10

1-isooctadecyloxycarbonyl-2-methyl-5(6)-nitrobenzimidazole was prepared from commercial isooctadecanol and 2-methyl-5(6) benzimidazole using the method of Example 6. The structure was confirmed by infrared and n.m.r. analysis.

The compound was evaluated as an extractant for copper and zinc using the procedure of Example 1, and the results are presented in Tables 1 and 2 respectively. The results show that the ligand has substantial affinity for copper.

Example 11

1-isooctadecyloxycarbonyl-5(6)-nitrobenzimidazole was prepared from commercial isooctadecanol and 5(6)-nitrobenzimidazole using the method of Example 6. The structure was confirmed by infrared and n.m.r. analysis.

The compound was evaluated as an extractant for copper and zinc using the procedure of Example 1, and the results are presented in Tables 1 and 2 respectively. The results show that the ligand has a good affinity for copper coupled with very low acid transfer.

Example 12

1-isooctadecyloxycarbonylimidazole was prepared as follows :

Isooctadecyl chloroformate (16.63 g) was added to a stirred solution of imidazole (11.3 g) in acetone (100 ml). On completion of the reaction the mixture was filtered and the solvent removed from the filtrate. The residual oil was dissolved in chloroform (100 ml) and washed with dilute hydrochloric acid followed by water. Drying with magnesium sulphate and removal of the solvent gave 15.43 g of a colourless oil. The structure of the product was confirmed by infrared and n.m.r. analysis.

The compound was evaluated as an extractant for copper and zinc using the procedure of Example 1, and the results are presented in Tables 1 and 2 respectively. The results show that the ligand has an extremely high affinity for both copper and zinc.

Example 13

3,9-Diethyl-tridecan-6-ol (a secondary alcohol) was converted to the corresponding chloroformate by reaction with phosgene, and the product was reacted with benzimidazole using the method of Example 1 to give 1-(3,9-diethyl-tridecyl-6-oxy carbonyl) benzimidazole. The compound was evaluated as an extractant for copper and zinc using the procedure of Example 1 and the results are presented in Tables 1 and 2 respectively. The results show that the ligand has good affinity for both zinc and copper coupled with a low acid transfer.

Example 14

1-(4-isooctadecyloxybenzoyl) benzimidazole was prepared from 4-isooctadecyloxybenzoyl chloride and benzimidazole using the method of Example 1. The compound was evaluated as an extractant for copper and zinc using the procedure of Example 1 and the results are presented in Tables 1 and 2 below. The results show that the ligand has a good affinity for both zinc and copper.

Example 15

1-(4-dodecylbenzene sulphonyl) benzimidazole was prepared by adding 4-dodecylbenzene sulphonyl chloride (17.25 g) in pyridine (10 ml) to a stirred mixture of benzimidazole (6.49 g) in pyridine (50 ml). After pouring into water (100 ml), the organic-soluble phase was extracted with petroleum ether, washed with 1N HCl and water, dried with magnesium sulphate and stripped of solvent of give 16.11 g of a viscous oil. The product was evaluated as an extractant for copper and zinc using the procedure of Example 1, and the results are presented in Tables 1 and 2 below. The results show that the ligand has good affinity for both copper and zinc coupled with a low acid transfer.

Table 1

| Example | HCl Molarity | $CaCl_2.2H_2O$ (g/l) | % Uptake from 0.1M $CuCl_2$ Copper | protonation |
|---|---|---|---|---|
| 1 | 0.1 | 250 | 42 | 0 |
| | 0.1 | 700 | 69 | 15 |
| | 1.0 | 250 | 57 | 8 |
| | 1.0 | 700 | 67 | 52 |
| 2 | 0.1 | 250 | 16 | 0 |
| | 0.1 | 700 | 55 | 8 |
| | 1.0 | 250 | 32 | 2 |
| | 1.0 | 700 | 63 | 62 |
| 3 | 0.1 | 700 | 31 | 1 |
| | 1.0 | 700 | 47 | 20 |
| 4 | 0.1 | 700 | 27 | 7 |
| | 1.0 | 250 | 30 | 2 |
| 5 | 0.1 | 700 | 49 | 21 |
| | 1.0 | 250 | 30 | 20 |
| | 1.0 | 700 | 59 | 43 |
| 6 | 0.1 | 700 | 27 | 18 |
| | 1.0 | 700 | 51 | 66 |
| 7 | 0.1 | 250 | 15 | 0 |
| | 0.1 | 700 | 67 | 0.5 |
| | 1.0 | 250 | 44 | 0 |
| | 1.0 | 700 | 67 | 7 |
| 8 | 0.1 | 700 | 49 | 26 |
| | 1.0 | 250 | 8 | 6 |

Table 1 (continued)

| Example | HCl Molarity | $CaCl_2.2H_2O$ (g/1) | % Uptake from 0.1M $CuCl_2$ Copper | protonation |
|---|---|---|---|---|
| 9. | 0.1 | 250 | 44 | 0 |
| | 0.1 | 700 | 75 | 2 |
| | 1.0 | 250 | 54 | 0 |
| | 1.0 | 700 | 74 | 43 |
| 10 | 1.0 | 700 | 40 | 34 |
| 11 | 0.1 | 700 | 45 | 0 |
| | 1.0 | 250 | 16 | 0 |
| | 1.0 | 700 | 49 | 1 |
| 12 | 0.1 | 250 | 88 | 0 |
| | 0.1 | 700 | 96 | 3 |
| | 1.0 | 250 | 92 | 1 |
| 13 | 0.1 | 250 | 25 | 0 |
| | 0.1 | 700 | 71 | 5 |
| | 1.0 | 250 | 34.5 | 0.5 |
| | 1.0 | 700 | 73 | 53 |
| 14 | 0.1 | 250 | 24 | 0 |
| | 0.1 | 700 | 79 | 13 |
| | 1.0 | 250 | 57 | 1.5 |
| | 1.0 | 700 | 72 | 73 |
| 15 | 0.1 | 250 | 8 | 0 |
| | 0.1 | 700 | 59 | 5 |
| | 1.0 | 250 | 16 | 0 |
| | 1.0 | 700 | 68 | 41 |

Table 2

| Example | HCl Molarity | $CaCl_2.2H_2O$ (g/l) | % Uptake from 0.6M $ZnCl_2$ Zinc | protonation |
|---|---|---|---|---|
| 1 | 0.01 | 176.5 | 66 | 0 |
| | 0.01 | 626.5 | 68 | 0 |
| | 0.1 | 626.5 | 75 | 41 |
| 2 | 0.01 | 176.5 | 24 | 0 |
| | 0.01 | 626.5 | 27 | 0 |
| | 0.1 | 626.5 | 54 | 28 |
| 3 | 0.01 | 626.5 | 2 | 0 |
| 4. | 0.01 | 626.5 | 2 | 0 |
| 5. | 0.01 | 176.5 | 11 | 0 |
| | 0.01 | 626.5 | 14 | 0 |
| | 0.1 | 626.5 | 76 | 42 |
| 6. | 0.01 | 626.5 | 1 | 0.5 |
| 7 | 0.01 | 176.5 | 12 | 0 |
| | 0.01 | 626.5 | 17 | 0 |
| | 0.1 | 626.5 | 36 | 15 |
| 8. | 0.01 | 176.5 | 7 | 0 |
| | 0.01 | 626.5 | 10 | 0 |
| | 0.1 | 626.5 | 74 | 44 |
| 9. | 0.01 | 176.5 | 52 | 0 |
| | 0.01 | 626.5 | 54 | 0.5 |
| | 0.1 | 626.5 | 74 | 29 |
| 10 | 0.01 | 626.5 | 1 | 0 |
| 11 | 0.01 | 626.5 | 2 | 0 |
| 12 | 0.01 | 176.5 | 89 | 0 |
| | 0.01 | 626.5 | 88 | 0 |
| | 0.1 | 626.5 | 96 | 46 |

Table 2 (continued)

| Example | HCl Molarity | $CaCl_2.2H_2O$ (g/l) | % Uptake from 0.6M $ZnCl_2$ Zinc | protonation |
|---|---|---|---|---|
| 13 | 0.01 | 176.5 | 46 | 0 |
| | 0.01 | 626.5 | 55 | 0 |
| | 0.1 | 626.5 | 73 | 32 |
| 14 | 0.01 | 176.5 | 68 | 0 |
| | 0.01 | 626.5 | 76 | 0 |
| | 0.1 | 626.5 | 87 | 41 |
| 15 | 0.01 | 176.5 | 22 | 0 |
| | 0.01 | 626.5 | 28 | 0 |
| | 0.1 | 626.5 | 58 | 27 |

**Claims**

1. A process for extracting metal values from aqueous solutions of metal salts containing halide or pseudo halide anions which comprises contacting the aqueous solution with a solution in a water-immiscible organic solvent of a N-substituted 1,3-diazole extractant of the formula :

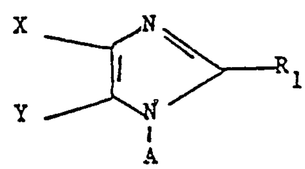

(I)

wherein :

$R_1$ is hydrogen or an optionally substituted lower alkyl group or an optionally substituted vinyl group ;

X and Y, which may be the same or different, are separately hydrogen, an optionally substituted hydrocarbon group, a halogen, a nitro group, a cyano group or a carboxylic ester group, or where X and Y both together with the two carbon atoms joining them form an optionally substituted aromatic or aliphatic cyclic group ; and

A is the group $—C = O \cdot OR_2$, $—C = O \cdot R_2$, $—SO_2R_2$ or $—C = O \cdot NH_3R_4$ wherein $R_2$ is a hydrocarbyl group containing from 5 to 36 carbon atoms and $R_3$ and

$R_4$ are separately hydrogen or a hydrocarbyl group, $R_3$ and $R_4$ together containing from 5 to 36 alkyl carbon atoms.

2. A process according to claim 1 wherein the extractant is a benzimidazole of formula :

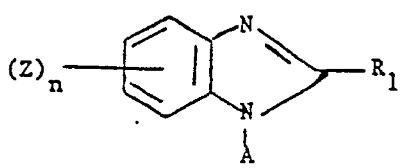

(II)

wherein $—(Z)_n$ represents n optional substituents Z and n is from 0 to 3 ;
or an imidazole of formula :

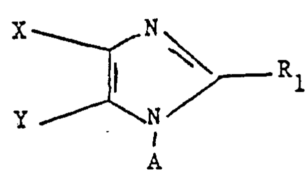

(III)

or a tetrahydrobenzimidazole of formula :

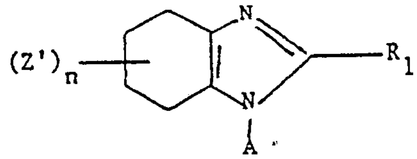

(IV)

wherein $(Z')_n$ represents n substituents Z' wherein n is from 0 to 3, Z (and Z') may separately be for example a hydrocarbyl group, halogen, a nitro group, a cyano group or a carboxylic ester group.

3. A process according to claim 2 wherein the extractant is a tetrahydrobenzimidazole, Z' is an hydrocarbyl group and n is 0, 1 or 2.

4. A process according to any one of claims 1 to 3 wherein $R_1$ is hydrogen.

5. A process according to any one of claims 1 to 4 wherein the total number of alkyl carbon atoms in all the substituents of the diazole is from 9 to 30.

6. A process according to claim 5 wherein the total number of alkyl carbon atoms is from 14 to 30.

7. A process according to any one of claims 1 to 6 wherein $R_2$ is an octyl, nonyl, decyl, dodecyl, tridecyl, pentadecyl, hexadecyl or octadecyl, cycloalkyl, alkylaryl or alkoxyaryl group.

8. A process according to any one of claims 1 to 7 wherein $R_2$ is a branched alkyl group or a mixture of branched alkyl groups.

9. A process according to claim 1 to 6 wherein the group $—OR_2$ is derived from an alcohol of the formula :

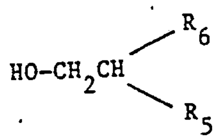

wherein $R_5$ and R-6 are both alkyl groups and $R_5$ contains two fewer carbon atoms than $R_6$.

10. A process according to claim 1 wherein the extractant is selected from the group consisting of :

1-isooctadecanoyl-5(6)-chlorobenzimidazole
1-isooctadecanoyl-5(6)-nitrobenzimidazole
1-isooctadecanoyl-2-methyl-5(6)-chlorobenzimidazole
1-isooctadecanoyl-2-methyl-benzimidazole
1-isooctadecyloxycarbonyl-2-methyl-5(6)-chlorobenzimidazole
1-isooctadecyloxycarbonyl-5(6)-chlorobenzimidazole
1-isooctadecyloxycarbonyl-2-methylbenzimidazole
1-isooctadecyloxycarbonylbenzimidazole
1-isooctadecyloxycarbonyl-2-methyl-5(6)-nitrobenzimidazole
1-isooctadecyloxycarbonyl-5(6)-nitrobenzimidazole
1-isooctadecyloxycarbonylimidazole
1-(4-isooctadecyloxybenzoyl) benzimidazole
1-(4-dodecylbenzenesulphonyl) benzimidazole.

11. A N-substituted 1,3-diazole compound of formula :

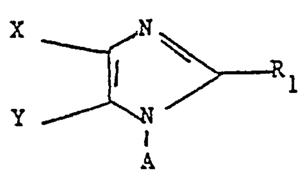

(I)

wherein :

$R_1$ is hydrogen or an optionally substituted lower alkyl group or an optionally substituted vinyl group ;

X and Y, which may be the same or different, are separately hydrogen, an optionally substituted hydrocarbon group, a halogen, a nitro group, a cyano group or a carboxylic ester group, or where X and Y both together with the two carbon atoms joining them form an optionally substituted aromatic or aliphatic cyclic group ; and

A is the group $—C = O \cdot OR_2$, $—C = O \cdot R_2$ or $—SO_2R_2$ wherein $R_2$ is an alkyl group containing from 9 to 24 carbon atoms.

## Patentansprüche

1. Verfahren zur Extraktion von Metallen aus wäßrigen Lösungen von Metallsalzen, die Halogenid oder Pseudohalogenid-anionen enthalten, bei welchem die wäßrige Lösung mit einer Lösung eines N-substituierten 1,3-Diazol-Extraktionsmittels der Formel

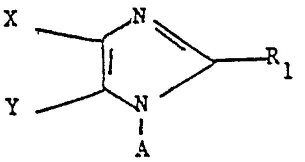

(I)

in einem mit Wasser unmischbaren organischen Lösungsmittel in Berührung gebracht wird, wobei in der Formel

$R_1$ für Wasserstoff oder eine gegebenenfalls substituierte Niederalkylgruppe oder eine gegebenenfalls substituierte Vinylgruppe steht ;

X und Y, welche gleich oder verschieden sein können, gesondert für Wasserstoff, eine gegebenenfalls substituierte Kohlenwasserstoffgruppe, ein Halogen, eine Nitrogruppe, eine Cyanogruppe oder eine Carbonsäureestergruppe stehen, oder worin X und Y gemeinsam mit den beiden sie verbindenden Kohlenstoffatomen eine gegebenenfalls substituierte aromatische oder aliphatische cyclische Gruppe bilden ; und

A für die Gruppe $—C{=}O{\cdot}OR_2$, $—C{=}O{\cdot}R_2$, $—SO_2R_2$ oder $—C{=}O{\cdot}NR_3R_4$ steht, worin $R_2$ eine Kohlenwasserstoffgruppe mit 5 bis 36 Kohlenstoffatomen bedeutet und $R_3$ und

$R_4$ gesondert Wasserstoff oder eine Kohlenwasserstoffgruppe bedeuten, wobei $R_3$ und $R_4$ gemeinsam 5 bis 36 Alkylkohlenstoffatome aufweisen.

2. Verfahren nach Anspruch 1, bei welchem das Extraktionsmittel ein Benzimidazol der Formel

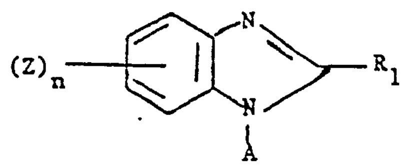

(II)

worin $—(Z)_n$ für n fakultative Substituenten Z steht und n für 0 bis 3 steht ;
oder ein Imidazol der Formel

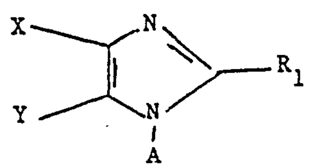

(III)

oder ein Tetrahydrobenzimidazol der Formel

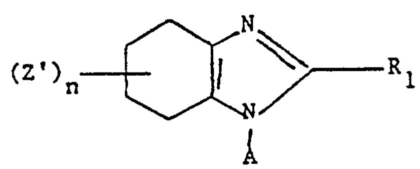

(IV)

ist, worin $(Z')_n$ für n Substituenten Z' steht und n für 0 bis 3 steht, wobei Z (und Z') gesondert für beispielsweise eine Kohlenwasserstoffgruppe, Halogen, eine Nitrogruppe, eine Cyanogruppe oder eine Carbonsäureestergruppe stehen können.

3. Verfahren nach Anspruch 2, bei welchem das Extraktionsmittel ein Tetrahydrobenzimidazol ist, Z' für eine Kohlenwasserstoffgruppe steht und n für 0, 1 oder 2 steht.

4. Verfahren nach einem der Ansprüche 1 bis 3, bei welchem $R_1$ für Wasserstoff steht.

5. Verfahren nach einem der Ansprüche 1 bis 4, bei welchem die Gesamtzahl der Alkylkohlenstoffatome in allen Substituenten des Diazols 9 bis 30 beträgt.

6. Verfahren nach Anspruch 5, bei welchem die Gesamtzahl der Alkylkohlenstoffatome 14 bis 30 beträgt.

7. Verfahren nach einem der Ansprüche 1 bis 6, bei welchem $R_2$ für eine Octyl-, Nonyl-, Decyl-, Dodecyl-, Tridecyl-, Pentadecyl-, Hexadecyl- oder Octadecyl- oder eine Cycloalkyl-, Alkylaryl- oder Alkoxyarylgruppe steht.

8. Verfahren nach einem der Ansprüche 1 bis 7, bei welchem $R_2$ für eine verzweigte Alkylgruppe oder ein gemisch von verzweigten Alkylgruppen steht.

9. Verfahren nach einem der Ansprûche 1 bis 6, bei welchem die Gruppe —$OR_2$ sich von einem Alkohol der Formel

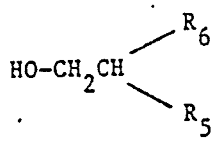

ableitet, worin $R_5$ und $R_6$ beide für Alkylgruppen stehen und $R_5$ zwei Kohlenstoffatome weniger als $R_6$ enthält.

10. Verfahren nach Anspruch 1, bei welchem das Extraktionsmittel ausgewählt ist aus :
1-Isooctadecanoyl-5(6)-chlorobenzimidazol
1-isooctadecanoyl-5(6)-nitrobenzimidazol
1-isooctadecanoyl-2-methyl-5(6)-chlorobenzimidazol
1-Isooctadecanoyl-2-methyl-benzimidazol
1-isooctadecyloxycarbonyl-2-methyl-5(6)-chlorobenzimidazol
1-Isooctadecyloxycarbonyl-5(6)-chlorobenzimidazol
1-Isooctadecyloxycarbonyl-2-methylbenzimidazol
1-Isooctadecyloxycarbonylbenzimidazol
1-Isooctadecyloxycarbonyl-2-methyl-5(6)-nitrobenzimidazol
1-Isooctadecyloxycarbonyl-5(6)-nitrobenzimidazol
1-isooctadecyloxycarbonylimidazol
1-(4-isooctadecyloxybenzoyl) benzimidazol
1-(4-dodecylbenzolsulphonyl) benzimidazol.

11. N-substituierte 1,3-Diazol-Verbindung der Formel

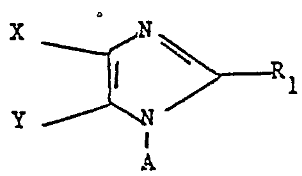

(I)

worin

$R_1$ für Wasserstoff oder eine gegebenenfalls substituierte Niederalkylgruppe oder eine gegebenenfalls substituierte Vinylgruppe steht ;

X und Y, welche gleich oder verschieden sein können, gesondert für Wasserstoff, eine gegebenenfalls substituierte Kohlenwasserstoffgruppe, ein Halogen, eine Nitrogruppe, eine Cyanogruppe oder eine Carbonsäureestergruppe stehen, oder worin X und Y gemeinsam mit den sie verbindenden beiden kohlenstoffatomen eine gegebenenfalls substituierte aromatische oder aliphatische cyclische Gruppe bilden ; und

A für die Gruppe —$C{=}O{\cdot}OR_2$, —$C{=}O{\cdot}R_2$ oder —$SO_2R_2$ steht, worin $R_2$ eine Alkylgruppe mit 9 bis 24 Kohlenstoffatomen bedeutet.

## Revendications

1. Procédé d'extraction de métaux de solutions aqueuses de sels métalliques contenant des anions halogénure ou pseudo-halogénure, qui consiste à mettre en contact la solution aqueuse avec une solution, dans un solvant organique non miscible à l'eau, d'un agent d'extraction du type 1,3-diazole N-substitué de formule :

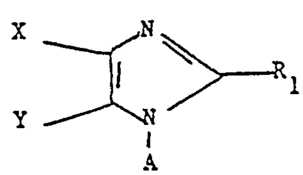

(I)

dans laquelle

$R_1$ représente l'hydrogène ou un groupe alkyle inférieur facultativement substitué, ou bien un groupe vinyle facultativement substitué ;

X et Y, qui peuvent être identiques ou différents, représentent séparément l'hydrogène, un groupe hydrocarboné facultativement substitué, un halogène, un groupe nitro, un groupe cyano ou un groupe ester carboxylique, ou bien X et Y forment ensemble, conjointement avec les deux atomes de carbone les reliant, un groupe aromatique ou cycloaliphatique facultativement substitué ; et

A représente un groupe $-C{=}O{\cdot}OR_2$, $-C{=}O{\cdot}R_2$, $-SO_2R_2$ ou $-C{=}O{\cdot}NR_3R_4$ dans lequel $R_2$ représente un groupe hydrocarbyle contenant 5 à 36 atomes de carbone et $R_3$ et

$R_4$ représentent séparément l'hydrogène ou un groupe hydrocarbyle, $R_3$ et $R_4$ contenant ensemble 5 à 36 atomes de carbone dans la portion alkyle.

2. Procédé suivant la revendication 1, dans lequel l'agent d'extraction est un benzimidazole de formule :

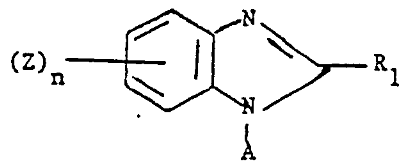

(II)

dans laquelle $-(Z)_n$ représente n substituants Z facultatifs et n a une valeur de 0 à 3 ;
ou un imidazole de formule :

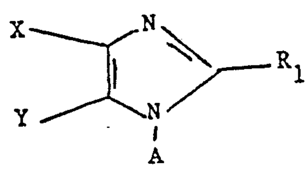

(III)

ou bien un tétrahydrobenzimidazole de formule

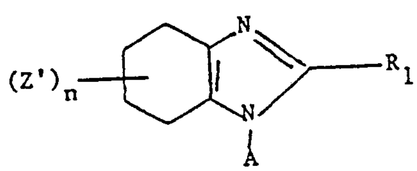

(IV)

dans laquelle $(Z')_n$ représente n substituants Z', n ayant une valeur de 0 à 3, Z (et Z') pouvant représenter séparément, par exemple, un groupe hydrocarbyle, un halogène, un groupe nitro, un groupe cyano ou un groupe ester carboxylique.

3. Procédé suivant la revendication 2, dans lequel l'agent d'extraction est un tétrahydrobenzimidazole, Z' est un groupe hydrocarbyle et n a la valeur 0, 1 ou 2.

4. Procédé suivant l'une quelconque des revendications 1 à 3, dans lequel $R_1$ représente l'hydrogène.

5. Procédé suivant l'une quelconque des revendications 1 à 4, dans lequel le nombre total d'atomes de carbone de la portion alkyle dans tous les substituants du diazole est compris dans l'intervalle de 9 à 30.

6. Procédé suivant la revendication 5, dans lequel le nombre total d'atomes de carbone de la portion alkyle est compris dans l'intervalle de 14 à 30.

7. Procédé suivant l'une quelconque des revendications 1 à 6, dans lequel $R_2$ représente un groupe octyle, nonyle, décyle, dodécyle, tridécyle, pentadécyle, hexadécyle ou octadécyle, cycloalkyle, alkylaryle ou alkoxyaryle.

8. Procédé suivant l'une quelconque des revendications 1 à 7, dans lequel $R_2$ représente un groupe alkyle ramifié ou un mélange de groupes alkyle ramifiés.

9. Procédé suivant les revendications 1 à 6, dans lequel le groupe $-OR_2$ est dérivé d'un alcool de formule :

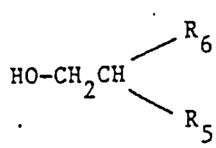

dans laquelle $R_5$ et $R_6$ représentent tous deux des groupes alkyle et $R_5$ contient deux atomes de carbone de moins que le groupe $R_6$.

10. Procédé suivant la revendication 1, dans lequel l'agent d'extraction est choisi dans le groupe comprenant :

le 1-isooctadécanoyl-5(6)-chlorobenzimidazole
le 1-isooctadécanoyl-5(6)-nitrobenzimidazole
le 1-isooctadécanoyl-2-méthyl-5(6)-chloro-benzimidazole
le 1-isooctadécanoyl-2-méthyl-benzimidazole
le 1-isooctadécyloxycarbonyl-2-méthyl-5(6)-chlorobenzimidazole
le 1-isooctadécyloxycarbonyl-5(6)-chlorobenzimidazole
le 1-isooctadécyloxycarbonyl-2-méthylbenzimidazole
le 1-isooctadécyloxycarbonylbenzimidazole
le 1-isooctadécyloxycarbonyl-2-méthyl-5(6)-nitrobenzimidazole
le 1-isooctadécyloxycarbonyl-5(6)-nitrobenzimidazole
le 1-isooctadécyloxycarbonylimidazole
le 1-(4-isooctadécyloxybenzoyl) benzimidazole
le 1-(4-dodécylbenzènesulfonyl) benzimidazole

11. 1,3-diazole N-substitué de formule :

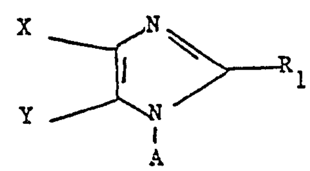

(I)

dans laquelle :

$R_1$ représente l'hydrogène ou un groupe alkyle inférieur facultativement substitué, ou bien un groupe vinyle facultativement substitué ;

X et Y, qui peuvent être identiques ou différents, représentent séparément l'hydrogène, un groupe hydrocarboné facultativement substitué, un halogène, un groupe nitro, un groupe cyano ou un groupe ester carboxylique, ou bien X et Y forment tous deux, conjointement avec les deux atomes de carbone les reliant, un groupe aromatique ou cycloaliphatique facultativement substitué ; et

A représente un groupe $—C{=}O{\cdot}OR_2$, $—C{=}O{\cdot}R_2$ ou $—SO_2R_2$ dans lequel $R_2$ représente un groupe alkyle contenant 9 à 24 atomes de carbone.